Europäisches Patentamt

European Patent Office

Office européen des brevets

(19)

(11) Numéro de publication: **0 332 537**
**A1**

(12) **DEMANDE DE BREVET EUROPEEN**

(21) Numéro de dépôt: **89400653.5**

(22) Date de dépôt: **08.03.89**

(51) Int. Cl.⁴: **A 61 L 9/03**

(30) Priorité: **08.03.88 FR 8802924**

(43) Date de publication de la demande:
**13.09.89 Bulletin 89/37**

(84) Etats contractants désignés:
**AT BE CH DE ES FR GB GR IT LI LU NL SE**

(71) Demandeur: **ATELIERS DE CONCEPTIONS ET D'INNOVATIONS INDUSTRIELLES**
**96, boulevard de la Mission Marchand**
**F-92400 Courbevoie (FR)**

(72) Inventeur: **Pozzo, Michel Lucien**
**32, Boulevard d'Inckerman**
**F-92200 Neuilly-sur-Seine (FR)**

(74) Mandataire: **Ahner, Francis et al**
**CABINET REGIMBEAU 26, avenue Kléber**
**F-75116 Paris (FR)**

(54) **Article de diffusion de substances volatiles notamment de parfum.**

(57) L'invention a pour objet un article consommable de diffusion dans l'atmosphère ambiante de substances volatiles actives, notamment de parfum, caractérisé en ce qu'il comporte un élément support en matière rétractable, notamment thermo-rétractable, dans lequel ladite substance est absorbée.

L'invention a également pour objet un procédé de diffusion de substances volatiles, notamment de parfum, à l'aide d'un article de ce type, ainsi qu'un dispositif pour la mise en oeuvre du procédé.

FIG_1

EP 0 332 537 A1

## Description

### ARTICLE DE DIFFUSION DE SUBSTANCES VOLATILES NOTAMMENT DE PARFUM

La présente invention concerne un article consommable de diffusion dans l'atmosphère ambiante de substances volatiles actives, notamment de parfum ainsi qu'un procédé de diffusion desdites substances et un dispositif support dudit article utile dans le procédé de diffusion.

Dans la présente demande, on entend par substances volatiles actives notamment des désodorisants, des insecticides, des bactéricides, des substances répulsives ou attirant les animaux et principalement des parfums.

La diffusion de parfums dans l'atmosphère fait appel à de nombreuses techniques. Parmi les plus simples, on citera notamment des dispositifs de diffusion consistant en des parfums liquides contenus dans un récipient tel qu'une coupelle placée à proximité d'une source de chaleur qui peut être notamment une ampoule électrique sous alimentation. Ce type de dispositif présente bien entendu certains dangers dans l'utilisation puisqu'il nécessite la manipulation de liquide inflammable au contact d'une source de chaleur relativement importante lorsqu'il s'agit d'une ampoule électrique par exemple.

On a proposé pour éviter ce genre de manipulations d'utiliser des éléments supports tels que des granulés poreux en matière plastique ou des pastilles de cellulose dans lesquels sont absorbées les substances à diffuser. Cependant, ces éléments sont en général relativement coûteux par rapport à l'utilisation qui en est faite et ne permettent pas en outre d'obtenir toujours une diffusion satisfaisante de la substance absorbée. Enfin, ces éléments ne sont pas consommables et ne permettent pas notamment de suivre, plus précisément, de visualiser l'état de chargement dudit élément pour prévoir son prochain rechargement, ou,le cas échéant et de préférence, son changement.

Il est en effet préférable que ce type de support n'ait pas à être rechargé par l'utilisateur mais qu'il soit plutôt consommable ou qu'il soit nécessaire de le changer lorsque toute la substance absorbée a été diffusée. En effet, il est bien connu que les mélanges contradictoires de parfums peuvent avoir l'effet le plus désagréable sur l'odeur obtenue, en fonction de certaines incompatibilités entre les parfums. Certaines erreurs de ce type pourraient donc advenir lors du rechargement de l'élément support à l'aide d'un parfum différent du premier parfum si des traces de celui-ci étaient toujours présentes.

A ce propos, on remarquera qu'il est nécessaire dans les dispositifs utilisant du parfum liquide contenu dans un récipient en contact avec une source de chaleur que le récipient soit soigneusement nettoyé entre chaque utilisation lorsque l'on change de substance parfumante.

Un but de la présente invention est donc de proposer un article diffusion qui assure une bonne diffusion de la substance volatile active à diffuser tout en palliant aux inconvénients précités des réalisations antérieures.

Pour ce faire, la présente invention a pour objet un article consommable de diffusion dans l'atmosphère ambiante de substances volatiles actives, notamment de parfum caractérisé en ce qu'il comporte un élément support en matière rétractable, notamment thermorétractable, ladite substance étant absorbée dans ledit élément.

Selon l'invention, ladite substance qui imprègne ledit élément est comme exsudée suite à la rétraction de l'élément jusqu'à obtenir une rétraction totale. Ce type de diffusion de la substance imprégnante est particulièrement efficace puisqu'il résulte le cas échéant de conditions physico-chimiques d'évaporation de la substance liées à la température, mais également des contraintes mécaniques provenant de la rétraction de l'élément support. Le matériau peut être thermorétractable, la rétraction sera alors consécutive à l'application d'une température. Toutefois, on peut utiliser selon l'invention un matériau rétractable à température ambiante au seul contact del'air ambiant,la proximité d'une source de chaleur n'étant plus obligatoire pour assurer la diffusion.On notera en outre que cette rétraction permet de suivreet de visualiser la consommation progressive de l'article de diffusion.

Dans un mode particulièrement approprié de réalisation de l'invention, ledit élément support est constitué de fibres textiles thermorétractables, de préférence non tissées, liées par une résine polymérique. Il aura par exemple la forme d'une pastille ou rondelle. Ce type de matériau permet de bien retenir la substance dans l'élément support qui fait en quelque sorte office d'éponge. L'article peut donc être utilisé sans risque de perte de liquide et donc d'accident, compte tenu de la proximité nécessaire d'une source de chaleur pour assurer la diffusion de ladite substance. L'absorption de la substance dans l'élément support dégage les fibres du liant et facilite la rétraction des fibres.

Ainsi, un type de support particulier, constitué de chlorofibres non tissées, liées par une résine de polychlorure de vinyl, se rétracte à partir de 120°C lorsqu'il n'est pas humidifié et seulement à partir de 60 à 80°C. lorsqu'il est humidifié par chargement avec une solution comprenant une substance parfumante.

Avantageusement, ladite substance est diluée dans un solvant peu ou non volatile lorsque la température est inférieure à la température de rétraction de l'élément support imprégné de ladite substance. Par exemple, la substance est diluée dans un solvant de type huileux tel que du type glycol notamment le dipropylèneglycol ou éthylènediglycol.

La dilution de la substance dans un solvant permet de bien imprégner l'élément support et donc de faciliter sa rétraction, mais aussi d'homogénéiser la répartition de ladite substance dans l'élément support et donc d'obtenir une diffusion de ladite substance dans l'atmosphère ambiante régulière dans le temps. L'utilisation d'un solvant non volatile offre l'avantage de pouvoir conserver l'article avant son utilisation dans un état d'humidification favorable à sa bonne rétraction.

Toutefois, de préférence, pour assurer la conservation de l'article dans les meilleures conditions, celui-ci sera conditionné dans une pochette en boîte étanche à l'air et à la lumière, par exemple en aluminium enduit de polypropylène. Il est en effet préférable que les parfums ne soient pas exposés à la lumière pour éviter leur dégradation chimique.

La présente invention a également pour objet un procédé de diffusion de substances volatiles, notamment de parfum, caractérisé en ce que ladite substance, absorbée dans un élément support en matière rétractable, notamment thermorétractable, se diffuse dans l'atmosphère suite à la rétraction dudit élément support.

Dans un mode préférentiel de réalisation du procédé, l'élément support se rétracte par chauffage, ce qui provoque également l'évaporation de ladite substance et donc sa diffusion.

La présente invention a donc pour objet un procédé de diffusion utilisant un article tel que caractérisé précédemment, notamment en plaçant ledit article à proximité d'une source de chaleur.

L'invention a également pour objet un dispositif support de l'article selon l'invention utile pour la mise en oeuvre du procédé de diffusion d'une substance volatile notamment de parfum, caractérisé en ce qu'il comporte essentiellement un élément du type tampon constitué par des fils de matériau métallique, entremêlés, puis compressés pour donner ledit élément de type tampon dans une forme adaptée à recevoir ledit article.

Ce dispositif peut être placé à proximité d'une source de chaleur, notamment d'une ampoule électrique par des moyens de fixation appropriés, par exemple directement sur l'ampoule au moyen d'une tige métallique dont une des extrémités est fixée au dispositif et l'autre qui est en forme de ressort à deux boucles, l'ampoule étant insérée entre les deux boucles du ressort.

La constitution de ce dispositif lui confère des effets techniques très intéressants. D'une part, il dissuade l'utilisateur d'y introduire directement un liquide de manière dangereuse, comme ce peut être le cas lorsque l'article est supporté par une coupelle. D'autre part et surtout, ce dispositif permet de pallier une série d'inconvénients rencontrés lorsque l'article, selon l'invention, est supporté par une coupelle à proximité d'une ampoule par exemple.

On observait en effet alors une rétraction trop importante et trop rapide de l'article due à une montée de la température trop importante à certains endroits de la coupelle, notamment les endroits les plus proches de l'ampoule. Du liquide était relargué suite à la rétraction de l'article, mais en quantité trop importante pour s'évaporer au fur et à mesure de son relargage. Il en résultait une condensation sur la coupelle avec des risques de contact de liquide avec l'ampoule.

En outre, la coupelle métallique était difficilement manipulable après utilisation en raison de sa température élevée.

Enfin, on observait sur la coupelle des traces résiduelles persistantes du parfum diffusé pouvant être à l'origine de mélanges hazardeux contradictoires avec des parfums différents diffusés ultérieurement.

Le dispositif selon l'invention présente une surface de contact du matériau constitutif du tampon avec l'air beaucoup plus grande que dans le cas d'un matériau plein comme la coupelle dans la mesure où l'air est en quelque sorte laminé, si bien que la température est mieux répartie et que l'on n'observe plus cette concentration ponctuelle de température qui provoquait de la condensation dans la coupelle.

De plus, en cas de condensation, la structure du dispositif retient le condensat qui est ensuite éliminé par évaporation suite au chauffage du dispositif.

Par ailleurs, ce dispositif peut être manipulé beaucoup plus facilement après utilisation dans la mesure où il se refroidit beaucoup plus rapidement que la coupelle quand il est chaud.

Enfin, il n'y a pas d'accumulation d'odeurs, c'est-à-dire de traces de parfums dans la texture de l'élément tampon, et l'on peut y adapter successivement des pastilles imbibées de parfums différents sans observer de mélange contradictoire de parfums.

Avantageusement, l'élément tampon est serti et revêtu d'une grille métallique protectrice.

La figure 1 représente un élément du type tampon selon l'invention.

La figure 2 représente une tige métallique permettant de maintenir le dispositif support de l'article selon l'invention à proximité d'une ampoule électrique.

D'autres avantages et caractéristiques de l'invention apparaîtront à l'aide de la description des modes de réalisation suivants.

On a utilisé comme élément support une rondelle ou pastille de nappe aiguilletée non tissée 100 % chlorofibre liée par une résine 100 % polychlorure de vinyl de référence 3 333-B de la société NORDLYS.

Ce matériau est ininflammable et devient thermorétractable à partir de 60 à 80° C lorsqu'il est imbibé par une solution parfumante. Il est en outre très léger (masse volumique de 65 à 75 kg/m3) et présente des propriétés d'absorption très remarquables.

Cette rondelle est imprégnée d'une dilution de substance parfumante dans du dipropylèneglycol à des concentrations en poids pouvant varier par exemple de 5 à 75 % suivant la nature et l'origine du parfum, notamment les parfums d'origine chimique étant plus puissants, ils seront utilisés à des concentrations plus faibles que les parfums d'origine végétale. On peut citer cependant pour des parfums naturels une concentration de 20 à 50 %.

Les rondelles ou pastilles proposées présentent par exemple un diamètre de 40 mm et une épaisseur de 4 mm. Ces rondelles peuvent se rétracter jusqu'à un diamètre d'environ 10 mm, ce qui représente un rapport de rétraction en volume de l'ordre de 1 à 10 et témoigne de l'effet de diffusion importante que l'on peut obtenir avec ces matériaux.

Pour son imprégnation, la rondelle est trempée dans la solution parfumante ; on peut dans une rondelle telle que décrite de 40 mm imbiber jusqu'à plusieurs grammes de solution.

Avantageusement la quantité de liquide pour ce type de rondelle ne dépassera pas 1 g de manière à ce que la quantité de liquide disponible suite à la rétraction ne dépasse pas la quantité de liquide évaporable par la chaleur dégagée par une ampoule électrique par exemple.

Cette rondelle peut être placée à proximité d'une source de chaleur (radiateur, flamme, ampoule électrique) et l'on peut ainsi diffuser dans l'atmosphère une substance parfumante pendant plus de 6 heures, étant entendu que l'utilisation de la rondelle peut être intermittente, celle-ci ne s'usant pas entre deux utilisations.

Toutes formes et tous volumes sont envisageables pour l'élément support. Une variante intéressante consiste à perforer la rondelle décrite précédemment pour augmenter ainsi la surface de contact entre la rondelle et l'air ambiant et partant, favoriser la diffusion de la substance parfumante absorbée dans la rondelle.

Avantageusement, l'article selon l'invention est placé à proximité d'une ampoule électrique en utilisant un dispositif support de l'article comportant un élément tampon constitué de tricot en acier inoxydable de marque KNITMESH (réf. 613A) compressé en forme d'un disque de diamètre sensiblement supérieur à celui de la pastille de chlorofibre décrit précédemment. Un élément tampon approprié pour une bonne rétention des condensats sera constitué de fils de diamètre de l'ordre d' 1/4 de mm, il sera compressé de manière à créer une perte de charge d'un fluide lors de son passage à travers l'élément tampon de l'ordre de 20 à 30 %, et l'épaisseur global de l'élément sera de l'ordre de 3 à 5 mm.

Ce tricot métallique, une fois compressé, peut être serti et revêtu d'une grille métallique pour éviter l'enchevêtrement des fibres rétractées de la pastille lors de l'utilisation avec la texture de l'élément tampon, enchevêtrement qui gêne le retrait de la pastille une fois celle-ci consommée. Si le tricot est bien compressé, la grille n'est pas nécessaire.

L'utilisation d'un matériau inoxydable permet son nettoyage à l'eau ou à tout autre détergent.

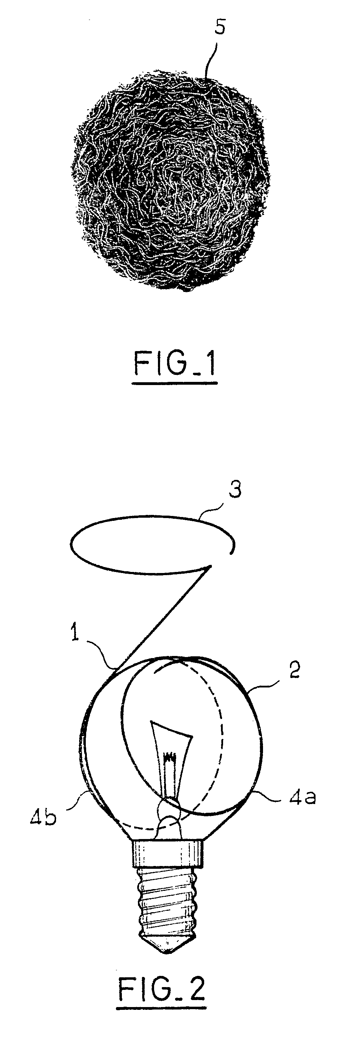

Sur la figure 1, on a un specimen brut de l'élément de type tampon constitué d'une multitude de fils de type métallique 5, inoxydables enchevêtrés et emmêlés, puis compressés. L'élément tampon tel que représenté n'est pas serti comme il le serait dans une présentation finie du dispositif de l'invention, tel que représenté figure 2.

Ce dispositif peut être fixé sur une ampoule électrique par l'intermédiaire d'une tige métallique 1 qui le maintient à proximité de l'ampoule 2. La tige métallique 1 se fixe à l'ampoule 2 en ce qu'une de ses extrémités est en forme de ressort à deux boucles 4a et 4b, l'ampoule étant insérée entre les deux boucles du ressort.

La tige est fixée à son autre extrémité 3 à l'élément tampon de la figure 1 en faisant adopter par exemple à ladite extrémité une forme en boucle que l'on enfonce dans la texture de l'élément tampon ou qui est sertie autour de l'élément tampon.

## Revendications

1. Article consommable de diffusion dans l'atmosphère ambiante de substances volatiles actives, notamment de parfum, caractérisé en ce qu'il comporte un élément support en matière rétractable, notamment thermorétractable, ladite substance étant absorbée dans ledit élément.

2. Article selon la revendication 1, caractérisé en ce que ledit élément support est constitué de fibres textiles thermorétractables, de préférence non tissées, liées par une résine polymérique.

3. Article selon l'une des revendications précédentes, caractérisé en ce que ledit élément support est constitué de chlorofibres non tissées, liées par une résine de polychlorure de vinyl.

4. Article selon l'une des revendications précédentes, caractérisé en ce que ladite substance est diluée dans un solvant peu ou non volatile lorsque la température est inférieure à la température de rétraction dudit élément support imprégné de ladite substance.

5. Article selon l'une des revendications précédentes, caractérisé en ce que ladite substance est diluée dans un solvant de type huileux tel que du type glycol notamment dipropylèneglycol.

6. Procédé de diffusion de substances volatiles, notamment de parfum, caractérisé en ce que ladite substance absorbée dans un élément support en matière rétractable, notamment thermorétractable, se diffuse dans l'atmosphère suite à la rétraction dudit élément support.

7. Procédé de diffusion selon la revendication 6, caractérisé en ce qu'on utilise un article selon l'une des revendications 1 à 5.

8. Procédé selon la revendication 7, caractérisé en ce qu'on place ledit article à proximité d'une source de chaleur, notamment d'une ampoule électrique.

9. Dispositif utile pour la mise en oeuvre du procédé selon l'une des revendications 7 et 8, caractérisé en ce qu'il comporte essentiellement un élément de type tampon constitué par des fils de matériau métallique entremêlés, puis compressés pour donner un élément de type tampon dans une forme adaptée à recevoir ledit article.

10. Dispositif selon la revendication précédente, caractérisé en ce que ledit élément de type tampon est du tricot de fil métallique ou d'acier inoxydable compressé.

11. Dispositif selon l'une des revendications 9 et 10, caractérisé en ce qu'il est fixé sur une ampoule électrique au moyen d'une tige métallique, laquelle est reliée sur le dispositif à une de ses extrémités et à .ladite ampoule par l'autre extrémité qui est en forme de ressort à deux boucles, lesdites deux boucles enserrant l'ampoule.

## FIG_1

## FIG_2

Office européen
des brevets

**RAPPORT DE RECHERCHE EUROPEENNE**

Numero de la demande

EP 89 40 0653

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 110 678 (UNILEVER)<br>* Revendications 1-5 *<br>--- | 1 | A 61 L 9/03 |
| A | US-A-4 184 099 (J.L. LINDAUER)<br>* Figure 1; revendication 1 *<br>--- | 1 | |
| A | US-A-2 220 583 (F.C. SCHNEBLY)<br>* Figure 4; revendications 1-3 *<br>----- | 1 | |

**DOMAINES TECHNIQUES RECHERCHES (Int. Cl.4)**

A 61 L

Le présent rapport a été établi pour toutes les revendications

| Lieu de la recherche | Date d'achèvement de la recherche | Examinateur |
|---|---|---|
| LA HAYE | 17-05-1989 | PELTRE CHR. |